# EUROPEAN PATENT APPLICATION

(11) **EP 3 703 104 A1**
(43) Date of publication of application: **02.09.2020**
(21) Application number: 19159604.8
(22) Date of filing: 27.02.2019
(51) Int. Cl.: H01J 61/44, H01J 61/35, H01J 61/46, H01J 61/48, C09K 11/77

(54) **A PHOSPHOR COMBINATION FOR A UV EMITTING DEVICE AND A UV GENERATING DEVICE UTILIZING SUCH A PHOSPHOR COMBINATION**

(71) Applicant: Xylem Europe GmbH, 8200 Schaffhausen (CH)
(72) Inventor: Broxtermann, Mike, 48149 Münster (DE); Jüstel, Thomas, 58455 Witten (DE); Salvermoser, Dr. Manfred, 32049 Herford (DE)
(74) Representative: Lenzing Gerber Stute

(57) **Abstract**

A UV emitting device, having
- at least one first phosphor which absorbs UV radiation of a wavelength shorter than 200 nm and emits UV radiation of a wavelength between 220 nm and 245 nm, and
- at least one second phosphor which absorbs UV radiation of a wavelength between 220 nm and 245 nm and emits UV radiation of a wavelength between 250 nm and 315 nm.

## Description

The present invention relates to a phosphor combination for a UV emitting device and to a UV generating device comprising such a phosphor combination.

A phosphor in this context is a chemical composition, which absorbs electromagnetic radiation of a certain energy and subsequently re-emits electromagnetic radiation exhibiting a different energy. Such phosphors are for example commonly known from fluorescent lamps. The term "phosphor" must not be understood as the chemical element Phosphorus. The term "phosphor combination" is to be understood as a combination of at least two phosphors, which can be applied in one or more layers, such as a mixture of two different phosphors, or a layered application of one layer of one phosphor covering a second layer of another phosphor.

UV-C emitting gas discharge lamps such as low pressure or medium pressure Hg discharge lamps are widely used for disinfection purposes in water and wastewater applications. They are also useful for so-called "advanced oxidation processes" for cracking highly persistent fluorinated or chlorinated carbons. Low pressure mercury gas discharge lamps emit UV-C mainly at 254 nm wavelength, which is radiated through the wall material of the lamps and sheath tubes, which are usually made of quartz. This part of the radiation is directly effective in damaging DNA of e.g. bacteria and viruses. Such lamps are very successfully applied in e.g. municipal water and wastewater treatment facilities.

On the other hand, environmental concerns lead to the need for mercury-free alternatives. Therefore, Xe excimer lamps have been developed, which emit a significant part of their radiation in a wavelength range of 172 nm ± 8 nm. This part of the electromagnetic spectrum is called "vacuum ultraviolet" (VUV). A large part of this high energy radiation is absorbed by the quartz body of the lamp and thus lost for the application.

Prior art documents disclose lamps for lighting purposes in which a combination of two phosphors is utilized, namely DE 101 29 630 A1, DE 103 24 832 A1 and US 6,982,046 B2. In all these documents, a first phosphor is used to absorb VUV radiation and emit UV radiation of a longer wavelength such as UV-C. A second phosphor is also provided in these lamps to absorb UV-C radiation and to emit radiation in the visible part of the electromagnetic spectrum. In this way, the part of the radiation energy that is emitted in the VUV region can be converted to visible light, thereby increasing the energetic efficiency of the lamp. However, visible light is not usable for disinfection purposes.

Disinfection by ultraviolet radiation requires lamps emitting in the UV-C part of the spectrum. Several phosphors have been proposed which convert radiation of 170 nm to 185 nm wavelength into longer wavelengths around 250 nm, for example in the documents US 6,734,631 B2, US 2005/0073239 A1, US 2012/0319011 A1, US 2008/0258601 A1, US 7,935,273 B2 and US 8,647,531 B2. These documents are herewith incorporated by reference. The phosphors proposed in the prior art documents have several drawbacks in the technical applications mentioned above.

First of all, many phosphors contain rare and expensive elements, making the use in large-scale installations too expensive. Furthermore, some of the compounds of the prior art do not show the desired long-term stability, which is necessary for example in municipal installations. What is more important is that these phosphors do not have a significant emission in the wavelength range between 255 nm and 265 nm, and especially that the Quantum Efficiency (QE), which describes the ratio between absorbed VUV photons and emitted UV-C photons, is not satisfactory for a good overall lamp efficiency.

Therefore, it is an object of the present invention to provide a novel UV-C and/or UV-B emitting device that is energy efficient and long-term stable, with an improved UV-C and/or UV-B emission spectrum with respect to specific applications such as disinfection or photochemistry.

It is also an object of the present invention to provide a novel phosphor combination, especially for mercury-free UV emitting devices, which improves on the deficiencies mentioned above. Furthermore, it is an object of the present invention to provide a UV generating device comprising such a phosphor.

This object is achieved by a UV generating device with the features of claim 1 and by a phosphor combination with the features of claim 10.

A UV emitting device, having
- at least one first phosphor which absorbs UV radiation of a wavelength shorter than 200 nm and emits UV radiation of a wavelength between 220 nm and 245 nm, and
- at least one second phosphor which absorbs UV radiation of a wavelength between 220 nm and 245 nm and emits UV radiation of a wavelength between 250 nm and 315 nm
can absorb VUV photons in the first phosphor and re-emit UV-C photons, and absorb UV-C photons in the second phosphor and re-emit UV-C and/or UV-B photons of a longer wavelength, such as 255 nm to 265 nm for disinfection purposes or other UV-C or UV-B wavelengths suitable for photochemical reactions, for example of 280 nm to 315 nm.

It is preferred that a gas discharge volume emitting, in operation, VUV radiation is provided, which is contained in a UV transparent vessel, the vessel having an inner surface and an outer surface, wherein the first phosphor and the second phosphor are either applied to the inner surface or to the outer surface of the vessel, or wherein the first phosphor is applied to the inside and the second phosphor is applied to the outside surface of the vessel.

Generally, these three options are available and can be chosen according to the requirements of the application.

If both layers of phosphor are applied to the inside surface of the vessel, then they are protected from adverse environmental influences. Since the VUV radiation is converted inside the vessel to longer wavelengths, common UV transparent quartz may be used, which is readily available and cost-effective.

If both layers are applied to the outside of the vessel, then the layers are hermetically separated from the interior of the vessel, which in some embodiments may contain chemical substances or elements, like mercury for example, which might degrade some phosphors upon coming in contact with them. In these cases, it would be preferred to apply both phosphors to the outer surface of the vessel. However, since the VUV radiation must pass through the vessel before reaching the first layer of phosphor, the vessel material must be VUV transparent, which makes it necessary to use special materials like synthetic quartz.

The third option is to apply the first layer of phosphor to the inside surface and the second layer to the outside surface of the vessel. In this case, only the first layer is in contact with the internal medium while the second layer of phosphor is protected from the internal medium. On the other hand, the first layer is protected from the environment, while the second layer is subject to environmental influences. In this embodiment, like in the first one, the VUV radiation is converted to longer wavelength UV already inside the vessel, therefore common quartz or other UV transparent materials may be used for producing the vessel.

Preferably, the first phosphor and the second phosphor are applied in the form of layers, wherein the first phosphor is positioned between the VUV emitting gas discharge volume and the second phosphor layer. This improves homogeneity of the coating and of the desired emission.

In a preferred embodiment, a coating is applied directly onto the inner surface and/or onto the outer surface of the vessel, wherein preferably the coating comprises Al₂O₃, MgO and/or SiO₂, and the layers comprising the phosphors are applied onto the coating. Such a coating and the application of the phosphors onto the coating improves the adhesion of the phosphors and thus the mechanical stability of the phosphor layers.

Manufacturing is facilitated if the vessel is a quartz tube.

Preferably the device is an excimer lamp, and more preferably a Xenon excimer UV lamp. These lamps have a suitable spectrum, long service life and good initial start-up behavior.

For environmental reasons, the device is preferably an excimer gas discharge lamp with a gas filling that is essentially free of mercury.

A phosphor combination for use in a UV-C emitting device also solves the problem, when the combination comprises
- at least one first phosphor which absorbs UV radiation of a wavelength shorter than 200 nm and emits UV radiation of a wavelength between 220 nm and 245 nm, and
- at least one second phosphor which absorbs UV radiation of a wavelength between 220 nm and 245 nm and emits UV radiation of a wavelength between 250 nm and 315 nm. It could be shown that the quantum efficiency of this combination is higher than the quantum efficiency of other phosphors, which convert VUV photons directly into longer wavelength photons with a wavelength between 250 nm and 315 nm. Furthermore, the first and second phosphors can be of a more cost-efficient and long-term stable kind.

Preferably the at least one first phosphor is one or more phosphor selected from the group comprising
CaSO₄:Pr,Na
SrSO₄:Pr,Na
LaPO₄:Pr
CaSO₄: Pb
LiLaP₄O₁₂:Pr
Y₂(SO₄)₃:Pr
LuPO₄:Pr
YPO₄:Pr
GdPO₄:Pr
NaMgPO₄:Pr
KSrPO₄:Pr
LiCaPO₄:Pr
LUPO₄:Bi
YPO₄:Bi
YBP₂O₈:Pr
YAlO₃:Pr
LaMgAl₁₁O₁₉:Pr
Ca₅(PO₄)₃F:Pr,K.

Also, the at least one second phosphor is advantageously one or more phosphor selected from the group comprising
Ca₉Lu(PO₄)₇: Pr
Ca₉Y(PO₄)₇: Pr
NaSrPO₄:Pr
NaCaPO₄:Pr
Sr₄Al₁₄O₂₅:Pr,Na
SrAl₁₂O₁₉:Pr,Na
CaLi₂SiO₄:Pr,Na
KCaPO₄:Pr
LuBO₃:Pr
YBO₃:Pr
Lu₂SiO₅:Pr
Y₂SiO₅:Pr
Lu₂Si₂O₇:Pr
CaZrO₃:Pr,Na
CaHfO₃:Pr,Na
Y₂Si₂O₇:Pr
Lu₃Al₅O₁₂:Bi,Sc
Lu₂SiO₅:Pr
Lu₃Al₃Ga₂O₁₂:Pr
Lu₃Al₄GaO₁₂:Pr
SrMgAl₁₀O₁₇:Ce,Na
Lu₃Al₅O₁₂:Pr
YBO₃:Gd
Lu₃Al₅O₁₂:Gd
Y₃Al₅O₁₂:Gd
LaMgAl₁₁O₁₉:Gd
LaAlO₃:Gd
YPO₄:Gd
GdPO₄:Nd
LaB₃O₆:Gd,Bi
SrAl₁₂O₁₉:Ce.

In a preferred embodiment, the first phosphor is YPO₄:Bi and the second phosphor is YBO₃:Pr.

A UV generating device with a UV radiation source comprising a phosphor combination as described above also solves the object of the invention, because a UV-C and/or UV-B source is provided with a relatively cost-effective phosphor combination having good VUV to UV-C and/or UV-B conversion efficiency at a desired target wavelength, and long-term stability.

Preferably the UV radiation source is a gas discharge lamp, especially an excimer gas discharge lamp, and it is preferred that the UV radiation source is an excimer gas discharge lamp with a gas filling that predominantly emits the second Xenon excimer continuum at VUV wavelengths around 172 nm. The gas filling may preferably contain more than 50% by volume of Xenon.

It is generally known how to produce phosphors of a given formula using wet chemistry. Generally, the compounds are used in batches in the form of oxides or phosphates in the desired molar ratio. These substances are then suspended in distilled water and, under stirring, H₃PO₄ is added and the suspension is stirred for several hours at ambient temperature. The suspension is then concentrated in an evaporator and dried. The solid residue is ground in a mortar. The powder can then be calcinated at high temperatures with exposure to air, for example up to 1000° C for 2-4 hours. After cooling to ambient temperature, the phosphor results as a solid. The phosphor can additionally be washed with distilled water, filtered off and dried in order to obtain a pure white powder.

A coating with the named phosphors can be applied to the lamp body by wet or dry deposition methods. These methods are known in the prior art.

In the following, an embodiment of the present invention is described in greater detail. Reference to the drawings is made, which show
- Fig. 1:: on the left side the Xe excimer emission spectrum (solid line) and superimposed with the photoluminescence excitation spectrum (dotted line), and on the right side the photoluminescence emission spectrum exhibited by YPO₄:Bi; and
- Fig. 2:: an emission spectrum of an Xe excimer discharge lamp with a double layer coating of YPO₄:Bi and YBO₃:Pr.

An Excimer discharge lamp comprising a double layer coating is disclosed, wherein the first layer comprises YPO₄:Bi (emission maximum 241 nm) and the second layer comprises YBO₃:Pr (emission maximum 265 nm).

Xe excimer discharge lamp bodies fabricated from high quality synthetic quartz were treated in a coating procedure which involves a four stepped spray coating of the lamp body surface with a first precoating layer of nanometer sized Al₂O₃ particles, a second covering layer of the UV-C emitting phosphor YPO₄:Bi (λ(Em.)max= 241 nm), a third covering layer of the UV-C/B emitting Phosphor YBO₃:Pr (λ(Em.)max= 265 nm) and a final protective capping layer of SiO2.

The base coating given by nanometer sized Al₂O₃ particles is applied onto the lamp vessel via spray coating utilizing a homogeneous 7.5 wt.-% dispersion of γ-Al₂O₃ (Trade name "AluC" provided by Evonik Industries AG, Essen, Germany) in iso-propanol. The coating was then applied in an airbrush spray-coating procedure involving continuous rotation of the lamp body along its longitudinal axis. The as coated lamp body is allowed to dry at room temperature for 20 minutes before it is further dried at 80°C for 1 h within a furnace.

The Al₂O₃ coated excimer lamp body is treated in another spray coating step involving a spray paint based on n-butylacetate as dispersing agent charged with 3 wt.% nitrocellulose (Type H7 provided by Hagedorn-NC GmbH, Osnabrück, Germany), 1 wt.-% Al₂O₃ (AluC, Evonik), 20 wt.% YPO₄:Bi (all wt.% values are related to the mass of n-butylacetate). In order to increase homogeneity, Al₂O₃ and YPO₄:Bi were gently mixed with 5 wt.% of an organic dispersing additive (Dysperbyk 110, provided by BYK-Chemie GmbH, Wesel, Germany), used relative to the summed up weight of Al₂O₃ and YPO₄:Bi, before dispersion in the homogeneous solution of nitrocellulose in iso-propanol. Homogeneity is achieved via agitation of the as prepared dispersion within a polyethylene bottle lying on a roller band for at least 2 hours. The coating was then applied in an airbrush spray coating procedure involving continuous rotation of the lamp body about its longitudinal axis.

The so coated lamp body is allowed to dry at room temperature for 1 hour. The drying is followed by a calcination at 500°C (30 min hold time) to bake out any organic components given by the applied YPO₄:Bi phosphor coating.

The Al₂O₃ precoated and YPO₄:Bi coated excimer lamp body is further treated in another spray coating involving a spray paint based on n-butylacetate as dispersing agent charged with 3 wt.-% nitrocellulose (Type H7, Hagedorn), 1 wt.-% Al₂O₃ (AluC, Evonik), 20 wt.-% YBO₃:Pr (all wt.-% values are related to the mass of n-butylacetate). In order to increase homogeneity, Al₂O₃ and YBO₃:Pr were gently mixed with 5 wt.-% of an organic dispersing additive (Dysperbyk 110, Byk), used relative to the summed up weight of Al₂O₃ and YBO₃:Pr before dispersion in the homogeneous solution of nitrocellulose in iso-propanol. Homogeneity is achieved via agitation of the as prepared dispersion within a polyethene bottle lying on a roller band for at least 2 hours. The coating was then applied in an airbrush spray coating procedure involving continuous rotation of the lamp body along its longitudinal axis. The as coated lamp body is allowed to dry at room temperature for 1 hour. The drying is followed by a calcination at 500 °C (30 min hold time) to bake out any organic components given by the applied YBO₃:Pr phosphor coating. The lamp coating procedure is finally completed by the application of a capping layer of SiO₂ utilizing a mixture of 1:1:0.25 mixture of ethanol, tetraethoxysilane in another, final airbrush spray coating procedure, continuously rotating of the lamp body along its longitudinal axis. The as coated lamp body is allowed to dry at room temperature for 1 hour followed by a final calcination at 500 °C (30 min hold time).

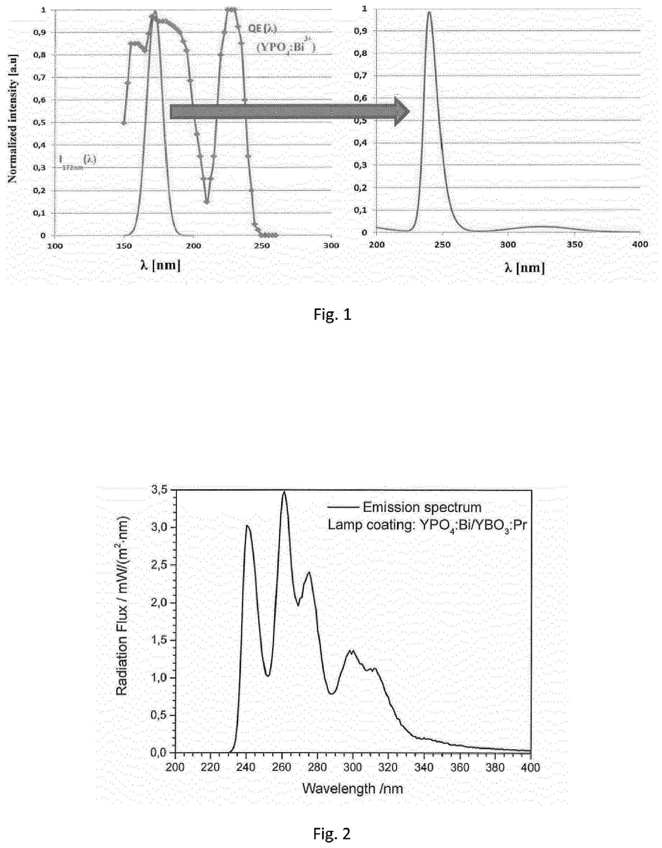

An Xe excimer lamp was produced in a known way using the so coated quartz tube as a tubular discharge vessel which contains the Xe gas filling as a discharge volume. The emission spectrum of an Xe excimer lamp with this coating is shown in Fig. 2.

## Claims

1. A UV emitting device, having
- at least one first phosphor which absorbs UV radiation of a wavelength shorter than 200 nm and emits UV radiation of a wavelength between 220 nm and 245 nm, and
- at least one second phosphor which absorbs UV radiation of a wavelength between 220 nm and 245 nm and emits UV radiation of a wavelength between 250 nm and 315 nm.

2. A UV emitting device according to claim 1, **characterized in that** a VUV emitting gas discharge volume is provided, which is contained in a UV transparent vessel, the vessel having an inner surface and an outer surface, wherein the first phosphor and the second phosphor are either
- both applied to the inner surface of the vessel or
- both to the outer surface of the vessel, or
- the first phosphor is applied to the inner surface of the vessel and the second phosphor is applied to the outer surface of the vessel.

3. A UV emitting device according claim 2, **characterized in that** the first phosphor and the second phosphor are applied in the form of layers, wherein the first phosphor is positioned between the discharge volume and the second phosphor layer.

4. A UV emitting device according to one of the preceding claims, **characterized in that** a coating is applied directly onto the inner surface and/or onto the outer surface of the vessel, wherein preferably the coating comprises Al₂O₃, MgO and/or SiO₂, and that the layers comprising the phosphors are applied onto the coating.

5. A UV emitting device according to one of the preceding claims, **characterized in that** the vessel is a quartz tube.

6. A UV emitting device according to one of the preceding claims, **characterized in that** the device is an excimer lamp.

7. A UV emitting device according to one of the preceding claims, **characterized in that** the device is a Xenon excimer UV lamp.

8. A UV emitting device according to one of the preceding claims, **characterized in that** the device is an excimer gas discharge lamp with a gas filling that is essentially free of mercury.

9. A phosphor combination for use in a UV-C and/or UV-B emitting device, **characterized in that** the combination comprises
- at least one first phosphor which absorbs UV radiation of a wavelength shorter than 200 nm and emits UV radiation of a wavelength between 220 nm and 245 nm, and
- at least one second phosphor which absorbs UV radiation of a wavelength between 220 nm and 245 nm and emits UV radiation of a wavelength between 250 nm and 315 nm.

10. A phosphor combination according to claim 9, **characterized in that** the at least one first phosphor is one or more phosphor selected from the group comprising
CaSO₄: Pr,Na
SrSO₄:Pr,Na
LaPO₄:Pr
CaSO₄: Pb
LiLaP₄O₁₂:Pr
Y₂(SO₄)₃:Pr
LuPO₄:Pr
YPO₄:Pr
GdPO₄:Pr
NaMgPO₄:Pr
KSrPO₄:Pr
LiCaPO₄:Pr
LUPO₄:Bi
YPO₄:Bi
YBP₂O₈:Pr
YAlO₃:Pr
LaMgAl₁₁O₁₉:Pr
Ca₅(PO₄)₃F:Pr,K.

11. A phosphor according to claim 9 or 10, **characterized in that** the at least one second phosphor is one or more phosphor selected from the group comprising
Ca₉Lu(PO₄)₇:Pr
Ca₉Y(PO₄)₇:Pr
NaSrPO₄:Pr
NaCaPO₄:Pr
Sr₄Al₁₄O₂₅:Pr,Na
SrAl₁₂O₁₉: Pr,Na
CaLi₂SiO₄:Pr,Na
KCaPO₄:Pr
LuBO₃:Pr
YBO₃:Pr
Lu₂SiO₅:Pr
Y₂SiO₅:Pr
Lu₂Si₂O₇:Pr
CaZrO₃:Pr,Na
CaHfO₃:Pr,Na
Y₂Si₂O₇:Pr
Lu₃Al₅O₁₂:Bi,Sc
Lu₂SiO₅:Pr
Lu₃Al₃Ga₂O₁₂:Pr
Lu₃Al₄GaO₁₂:Pr
SrMgAl₁₀O₁₇:Ce,Na
Lu₃Al₅O₁₂:Pr
YBO₃:Gd
Lu₃Al₅O₁₂:Gd
Y₃Al₅O₁₂:Gd
LaMgAl₁₁O₁₉:Gd
LaAlO₃:Gd
YPO₄:Gd
GdPO₄:Nd
LaB₃O₆:Gd,Bi
SrAl₁₂O₁₉:Ce.

12. A phosphor according to claim 9, **characterized in that** the first phosphor is YPO₄:Bi and the second phosphor is YBO₃:Pr.
